# EUROPÄISCHE PATENTSCHRIFT

(11) **EP 3 490 504 B1**
(45) Veröffentlichungstag und Bekanntmachung des Hinweises auf die Patenterteilung: **09.09.2020**
(21) Anmeldenummer: 17745724.9
(22) Anmeldetag: 31.07.2017
(51) Int. Cl.: A61F 2/38, A61F 2/30, A61F 2/32

(54) **IMPLANTAT UND GELENKIMPLANTAT**
IMPLANT AND JOINT IMPLANT
IMPLANT ET IMPLANT ARTICULAIRE

(30) Priorität: 29.07.2016 DE 102016114059
(43) Veröffentlichungstag der Anmeldung: 05.06.2019
(73) Patentinhaber: Aesculap AG, 78532 Tuttlingen (DE)
(72) Erfinder: HAGEN, Thomas, 78532 Tuttlingen (DE); SCHULZ, Thomas, 01877 Schmölln-Putzkau (DE); MANN, Sacha T. W., 35463 Fernwald (DE); GRUPP, Thomas, 78588 Denkingen (DE)
(74) Vertreter: Hoeger, Stellrecht & Partner Patentanwälte mbB
(86) Internationale Anmeldenummer: PCT/EP2017/069258
(87) Internationale Veröffentlichungsnummer: WO 2018/020048

(56) Entgegenhaltungen:
- EP-A1- 1 133 957
- CA-A1- 2 533 534
- US-A1- 2007 224 242
- US-A1- 2010 211 180

## Beschreibung

Die vorliegende Erfindung betrifft ein Implantat mit einem in eine Knochenkavität einführbaren Schaft, welcher Schaft aus einem körperunverträglichen Kunststoff ausgebildet ist und mindestens eine Knochenkontaktfläche definiert, wobei die Knochenkontaktfläche mit einer ersten körperverträglichen Knochenkontaktschicht versehen oder beschichtet ist oder eine körperverträgliche Knochenkontaktschicht trägt.

Ferner wird durch die vorliegende Erfindung ein Gelenkimplantat mit einer ersten Gelenkkomponente und mindestens einer zweiten, mit der ersten Gelenkkomponente zusammenwirkenden Gelenkkomponente.

Gelenkimplantate der eingangs beschriebenen Art werden beispielsweise in Form von Kniegelenkendoprothesen eingesetzt, um degenerierte oder deformierte Kniegelenke von Menschen zu ersetzen. Eine solche Kniegelenkendoprothese umfasst mindestens zwei Gelenkkomponenten, nämlich eine Tibiakomponente und eine Femurkomponente, die miteinander zusammenwirken zur Ausbildung eines künstlichen Kniegelenks. Die beiden Gelenkkomponenten sind dabei insbesondere in Form eines Implantats der eingangs beschriebenen Art ausgebildet.

Beispielsweise aus der US 4,209,861 ist eine Kniegelenkendoprothese mit einer Tibiakomponente bekannt, die vollständig aus einem Kunststoff ausgebildet ist, beispielsweise einem Polymer hoher Dichte wie UHMWPE.

Es hat sich in den letzten Jahrzehnten gezeigt, dass Implantate aus Kunststoff, insbesondere Implantate aus UHMWPE, nicht in direkten Kontakt mit Knochen kommen sollten. UHMWPE ist ein hydrophober Werkstoff und bietet damit für Knochengewebe sehr ungünstige Bedingungen, um in die Oberfläche des Implantats einzuwachsen. Daher entstehen dauerhaft Mikrobewegungen an der Grenzfläche zwischen Knochengewebe und der Oberfläche des Implantats, die zu einer Bindegewebsdeformation sowie zu einem Abbau des Kunststoffs am Implantat führen können. Um dieses Problem zu vermeiden, werden Tibiakomponenten von Kniegelenkendoprothesen, die vollständig aus einem Kunststoff ausgebildet sind, mit Knochenzement fixiert. Es bildet sich dabei ein Zementmantel beziehungsweise eine Zementschicht zwischen der Knochenkontaktfläche des Implantats und dem Knochengewebe. Damit dient der Knochenzement einerseits zur Fixierung des Implantats im beziehungsweise am Knochen und gleichzeitig auch als Trennschicht zwischen dem Kunststoff und dem Knochengewebe.

Ein Problem bei der Zementierung von Schäften von Tibiakomponenten, die vollständig aus Kunststoff ausgebildet sind, ist, dass es am Ende des Schafts zu Spannungsspitzen kommen kann. Diese treten bei einer Implantation der Tibiakomponente, welche im Schaftbereich nicht einzementiert wird, nicht auf. Die beschriebenen Spannungsspitzen können im ungünstigsten Fall zu einem Bruch der Tibia, also des den Schaft umgebenden Knochens, führen.

Aus der US 2010/0211180 A1 und der US 2007/0224242 A1 sind medizinische Vorrichtungen bekannt, die mit tetraedrisch amorphem Kohlenstoff beschichtet sind. Implantate mit Oberflächen zum Einwachsen von Knochen sind in der EP 1 133 957 A1 beschrieben. Poröse Strukturen in Form von nicht-zufälligen Schäumen sind in der CA 2 533 534 A1 offenbart.

Es ist daher eine Aufgabe der vorliegenden Erfindung, ein Implantat oder ein Gelenkimplantat der eingangs beschriebenen Art so zu verbessern, dass mindestens teilweise auf eine Knochenzementfixierung verzichtet werden kann.

Diese Aufgabe wird bei einem Implantat der eingangs beschriebenen Art erfindungsgemäß dadurch gelöst, dass der Schaft des Implantats bestimmungsgemäß ohne Knochenzement in der Knochenkavität verankerbar ist und dass die erste Knochenkontaktschicht vollständig geschlossen ausgebildet ist.

Die vorgeschlagene Weiterbildung eines bekannten Implantats ermöglicht es insbesondere, dieses ohne den Einsatz von Knochenzement an oder in einem Knochen festzulegen. Insbesondere kann der Schaft, dessen mindestens eine Knochenkontaktfläche mit der ersten körperverträglichen Knochenkontaktschicht versehen oder beschichtet ist oder diese trägt, direkt mit Knochen oder Knochengewebe in Kontakt gebracht werden, ohne dass die oben beschriebenen unerwünschten Folgen auftreten. Man kann also insbesondere auf die Zementierung des Schafts verzichten, so dass auch die oben beschriebenen Spannungsspitzen dann nicht auftreten und das Risiko für einen Bruch der Tibia deutlich verringert werden kann. Selbstverständlich kann das Implantat auch Flächen aufweisen, die mit Knochen in Kontakt treten können. Diese können, wenn sie nicht mit einer körperverträglichen Knochenkontaktschickt versehen sind, beispielweise durch eine Knochenzementschicht vom Knochengewebe getrennt werden. Insbesondere kann eine Tibiaplatte so auf einer entsprechend präparierten, in Richtung des Femur weisenden Knochenfläche der Tibia mittels Knochenzement fixiert werden. Gemäß der Erfindung ist der Schaft des Implantats bestimmungsgemäß ohne Knochenzement in der Knochenkavität verankerbar. Bestimmungsgemäß bedeutet in diesem Fall, dass der Schaft aus einem Material ausgebildet oder mit einem Material beschichtet ist, welches einen direkten Kontakt mit Knochengewebe ermöglicht, ohne dass die oben beschriebenen unerwünschten Nebeneffekte auftreten, wie sie von Kunststoffimplantaten bekannt sind, bei denen Kunststoff in direktem Kontakt mit Knochengewebe steht. Ein derart ausgebildeter Schaft kann durch die körperverträgliche Knochenkontaktschicht also ohne Knochenzement in der Knochenkavität verankert werden, und zwar ohne die Gefahr, dass es zu einer Bindegewebsdeformation oder zur teilweisen Zerstörung des aus Kunststoff ausgebildeten Schafts kommen kann. Gemäß der Erfindung ist die erste Knochenkontaktschicht vollständig geschlossen ausgebildet. Auf diese Weise kann insbesondere sichergestellt werden, dass Knochengewebe nicht in Kontakt mit dem Kunststoff kommt, aus dem das Implantat ausgebildet ist.

Vorteilhaft ist es, wenn das Implantat einen Grundkörper umfasst und wenn sich der Schaft von einer Unterseite des Grundkörpers weg erstreckt. Der Grundkörper kann beispielsweise in Form einer Tibiaplatte mit Gleitflächen zur Anlage an entsprechende Gleitflächen einer Femurkomponente ausgebildet sein. Der sich vom Grundkörper weg erstreckende Schaft dient zu einer optimierten Verankerung des Implantats am Knochen.

Um eine Befestigung des Implantats am Knochen weiter zu verbessern, kann insbesondere vorgesehen sein, dass der Grundkörper eine Grundkörperknochenkontaktfläche aufweist zum Anlegen an eine präparierte Knochenfläche. Mit anderen Worten kann also nicht nur der Schaft mit Knochengewebe in Kontakt kommen, sondern auch der Grundkörper oder ein anderer Teil des Implantats. Ist die Grundkörperknochenkontaktfläche nicht mit einer körperverträglichen Knochenkontaktschicht versehen, kann das Implantat zur Vermeidung der Nachteile eines Kontakts von Kunststoffen mit Knochengewebe mittels Knochenzement am Knochen befestigt werden.

Ferner kann es vorteilhaft sein, wenn die Grundkörperknochenkontaktfläche mit einer zweiten körperverträglichen Knochenkontaktschicht versehen ist oder eine zweite körperverträgliche Knochenkontaktschicht trägt. Diese Weiterbildung gestattet es insbesondere, das Implantat vollständig ohne Knochenzement am Knochen zu verankern. Die oben beschriebenen, unerwünschten Nachteile eines Kontakts von Kunststoffen und Knochengewebe können so auf einfache Weise vermieden werden.

Ferner ist es günstig, wenn der Grundkörper bestimmungsgemäß ohne Knochenzement am Knochen verankerbar ist. Bestimmungsgemäß bedeutet auch hier, dass der Grundkörper aus einem Material ausgebildet oder mit einem Material beschichtet ist, welches einen direkten Kontakt mit Knochengewebe ermöglicht, ohne dass die oben beschriebenen unerwünschten Nebeneffekte auftreten, wie sie von Kunststoffimplantaten bekannt sind, bei denen Kunststoff in direktem Kontakt mit Knochengewebe steht.

Besonders einfach ausbilden lässt sich das Implantat, wenn die erste Knochenkontaktschicht und die zweite Knochenkontaktschicht identisch ausgebildet sind. Insbesondere können sie in einem Fertigungsschritt aufgebracht werden, beispielsweise wenn das Implantat einstückig, insbesondere monolithisch ausgebildet ist.

Vorteilhaft ist es, wenn die erste und/oder die zweite Knochenkontaktschicht eine Dicke in einem Bereich von etwa 1 µm bis etwa 1000 µm aufweisen. Insbesondere können sie eine Dicke in einem Bereich von etwa 5 µm bis etwa 700 µm aufweisen. Eine Knochenkontaktschicht mit einer Dicke im angegebenen Bereich auszubilden, hat den Vorteil, dass das Implantat trotz seiner vorteilhaften Eigenschaft, nämlich ohne Knochenzement implantiert werden zu können, besonders leicht ausgebildet werden kann. Die erste und/oder die zweite Knochenkontaktschicht sind vorzugsweise mindestens so dick, dass ein direkter Knochenkontakt zwischen dem Kunststoff, aus dem das Implantat ausgebildet ist, und Knochengewebe sicher vermieden werden kann.

Um das Einwachsen von Knochengewebe beziehungsweise das Anwachsen von Knochengewebe an das Implantat zu verbessern, ist es günstig, wenn die erste und/oder die zweite Knochenkontaktschicht rau ausgebildet sind. Vorzugsweise weisen sie eine Rauheit in einem Bereich von etwa 0,5 µm bis etwa 10 µm auf.

Um die erste und/oder die zweite Knochenkontaktschicht mit einer gewünschten Rauheit ausbilden zu können bei gleichzeitig einfacher Herstellung des Implantats, ist es vorteilhaft, wenn die Knochenkontaktfläche und/oder die Grundkörperknochenkontaktfläche eine Rauheit in einem Bereich von etwa 0,5 µm bis etwa 10 µm aufweisen. Die jeweiligen Oberflächen des Implantats, die dazu bestimmt sind, mit Knochengewebe in Kontakt zu treten, können dann auf einfache Weise mit einer dünnen Knochenkontaktschicht beschichtet werden, wobei dann die Rauheit der Knochenkontaktfläche und/oder der Grundkörperknochenkontaktfläche beibehalten oder im Wesentlichen beibehalten werden können. Dies kann beispielsweise durch Aufbringen der Knochenkontaktschichten mittels Kaltgasspritzen und/oder durch physikalische und/oder durch chemische Gasphasenabscheidung erfolgen.

Günstig ist es, wenn die erste und/oder die zweite Knochenkontaktschicht aus mindestens einem Metall und/oder aus einem körperverträglichen Kunststoff und/oder aus einer Keramik und/oder aus mindestens einer knochenmineralischen Substanz ausgebildet sind. Abhängig vom Kunststoff, aus dem das Implantat ausgebildet ist, kann dann das passende Material ausgebildet werden, um die erste und/oder zweite Knochenkontaktschicht auszubilden.

Vorzugsweise ist das mindestens eine Metall Titan oder enthält Titan. Es kann also insbesondere auch eine Legierung sein, die Titan und andere körperverträgliche Metalle enthält.

Vorteilhafterweise ist oder enthält der körperverträgliche Kunststoff Polyetheretherketon (PEEK). PEEK ist optimal geeignet, um leichte und dennoch körperverträgliche Implantate auszubilden, insbesondere auch nicht körperverträgliche Kunststoffe zu beschichten.

Ferner kann es günstig sein, wenn die knochenmineralische Substanz Hydroxylapatit ist. Hydroxylapatit kommt in Lebewesen vor und entsteht insbesondere im menschlichen Körper durch Biomineralisation. Es ist in Knochen zu einem Anteil von etwa 40 % enthalten. So kann ein besonders knochenkompatibler Kontakt zwischen dem Knochen und dem Implantat ausgebildet werden.

Eine Beschichtung des aus Kunststoff ausgebildeten Implantats kann auf einfache Weise erreicht werden, wenn die erste und/oder die zweite Knochenkontaktschicht durch Kaltgasspritzen und/oder durch physikalische und/oder durch chemische Gasphasenabscheidung ausgebildet sind. Insbesondere lassen sich so dünne Knochenkontaktschichten ausbilden, beispielweise in den oben angegebenen Dickenbereichen.

Vorzugsweise ist die zweite Knochenkontaktschicht vollständig geschlossen ausgebildet. Auf diese Weise kann insbesondere sichergestellt werden, dass Knochengewebe nicht in Kontakt mit dem Kunststoff kommt, aus dem das Implantat ausgebildet ist.

Günstig kann es ferner sein, wenn das Implantat bis auf die erste und/oder die zweite Knochenkontaktschicht vollständig aus dem körperunverträglichen Kunststoff ausgebildet ist. Dies ermöglicht es insbesondere, das Implantat beziehungsweise einen dieses definierenden Implantatgrundkörper aus einem Kunststoff herzustellen, welcher die gewünschten mechanischen Eigenschaften aufweist, die zur Ausbildung des Implantats benötigt werden. Es muss insbesondere keine Rücksicht darauf genommen werden, ob dieser Kunststoff körperverträglich ist oder nicht, da durch die erste und/oder die zweite Knochenkontaktschicht ein direkter Kontakt zwischen dem körperunverträglichen Kunststoff und Knochengewebe des Patienten vermieden werden kann.

Insbesondere zur Ausbildung von Gleitflächen bei Gelenkimplantaten ist es vorteilhaft, wenn der nicht körperverträgliche Kunststoff Polyethylen oder Polyethylen mit ultrahohem Molekulargewicht (UHMWPE) ist. So lassen sich insbesondere Gleitflächen integral oder monolithisch mit dem Implantat ausbilden. Die Zahl der erforderlichen Teile für eine Endoprothese kann somit auf einfache Weise minimiert werden.

Günstigerweise ist das Implantat einstückig ausgebildet. Insbesondere kann es monolithisch ausgebildet sein. Unter einem Implantat ist hier insbesondere ein Implantatgrundkörper zu verstehen, welcher die erste und/oder die zweite Knochenkontaktschicht aufweist.

Günstig ist es, wenn das Implantat in Form einer ersten Gelenkkomponente eines künstlichen Gelenks oder Gelenkimplantats ausgebildet ist und mindestens eine Gelenkfläche aufweist. Mit einer zweiten, korrespondierend ausgebildeten Gelenkkomponente kann so auf einfache Weise ein künstliches Gelenk ausgebildet werden. Insbesondere kann die mindestens eine Gelenkfläche, es können auch zwei, drei oder mehr sein, integral oder monolithisch mit dem Implantat beziehungsweise dem Implantatgrundkörper ausgebildet werden.

Vorzugsweise ist die mindestens eine Gelenkfläche durch den körperunverträglichen Kunststoff ausgebildet. Der Kunststoff kann insbesondere so gewählt werden, dass seine Eigenschaften zur Ausbildung einer möglichst reibungs- und verschleißarmen Gelenkfläche in vorteilhafter Weise nutzbar sind. Insbesondere kann hier UHMWPE zum Einsatz kommen.

Zur Ausbildung einer Kniegelenkendoprothese ist es insbesondere vorteilhaft, wenn die erste Gelenkkomponente in Form einer Tibiakomponente ausgebildet ist. Sie kann alternativ auch in Form einer Femurkomponente ausgebildet sein. Auch andere Gelenke lassen sich so künstlich herstellen, beispielsweise künstliche Schultergelenke, künstliche Sprunggelenke oder auch künstliche Hüftgelenke.

Die eingangs gestellte Aufgabe wird ferner bei einem Gelenkimplantat der eingangs beschriebenen Art erfindungsgemäß dadurch gelöst, dass die erste und/oder die mindestens eine zweite Gelenkkomponente in Form eines der oben beschriebenen vorteilhaften Implantate ausgebildet sind. Das Gelenkimplantat insgesamt weist dann mindestens teilweise auch die oben im Zusammenhang mit bevorzugten Ausführungsformen von Implantaten beschriebenen Vorteile auf.

Günstig ist es, wenn das Gelenkimplantat in Form einer Kniegelenkendoprothese ausgebildet ist, wenn die erste Gelenkkomponente in Form einer Tibiakomponente und wenn die zweite Gelenkkomponente in Form einer Femurkomponente ausgebildet ist. So kann auf einfache Weise eine Kniegelenkendoprothese ausgebildet werden, welche mindestens eine, insbesondere zwei Gelenkkomponenten umfasst, die in Form von oben beschriebenen vorteilhaften Implantaten ausgebildet sind. Derartige Kniegelenkendoprothesen sind dauerhaft stabil und optimal körperverträglich. Ferner können sie ein minimales Gewicht aufweisen, was eine Gewöhnung des Patienten an das künstliche Gelenk verbessert.

Die nachfolgende Beschreibung bevorzugter Ausführungsformen der Erfindung dient im Zusammenhang mit den Zeichnungen der näheren Erläuterung. Es zeigen:
- Figur 1:: eine Seitenansicht eines Gelenkimplantats in Form einer Kniegelenkendoprothese;
- Figur 2:: eine perspektivische Ansicht der in Figur 1 dargestellten Tibiakomponente der Kniegelenkendoprothese;
- Figur 3:: eine weitere perspektivische Ansicht der Tibiakomponente aus Figur 2; und
- Figur 4:: ein weiteres Ausführungsbeispiel einer Tibiakomponente einer Kniegelenkendoprothese.

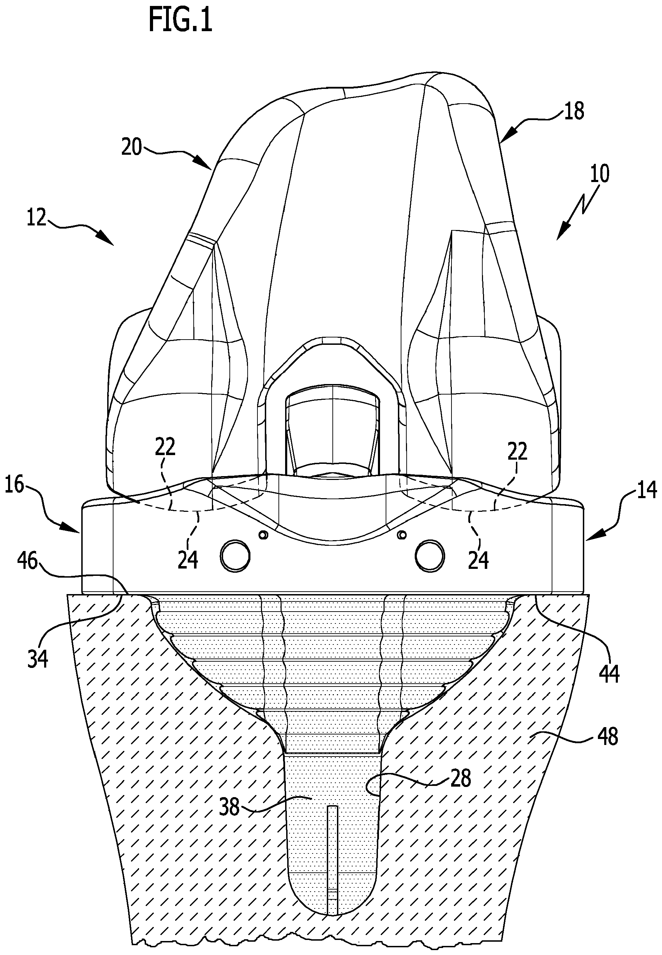

In Figur 1 ist beispielhaft ein mit dem Bezugszeichen 10 bezeichnetes Gelenkimplantat in Form einer Kniegelenkendoprothese 12 schematisch dargestellt.

Das Gelenkimplantat 10 umfasst eine erste Gelenkkomponente 14 in Form einer Tibiakomponente 16 sowie eine zweite Gelenkkomponente 18 in Form einer Femurkomponente 20.

Die erste Gelenkkomponente 14 weist zwei Gelenkflächen 22 auf, die korrespondierend zu zwei Gelenkflächen 24 der zweiten Gelenkkomponente 18 ausgebildet sind. Die Gelenkflächen 22 und 24 liegen bestimmungsgemäß insbesondere aneinander an und können optional aneinander abgleiten und/oder aneinander abrollen zur Ausbildung eines künstlichen Gelenks.

Die beiden Gelenkkomponenten 14 und 18 werden nachfolgend auch abkürzend als Implantat 26 bezeichnet.

Das Vorsehen von Gelenkflächen 22 beziehungsweise 24 am Implantat 26 ist nicht zwingend, allenfalls wenn zwei zusammenwirkende Implantate 26 zur Ausbildung eines Gelenkimplantats 10 vorgesehen werden. Denkbar ist es also insbesondere auch, ein einzelnes Implantat 26 als teilweisen Ersatz eines Knochens im Körper eines Menschen oder eines Tieres zu implantieren.

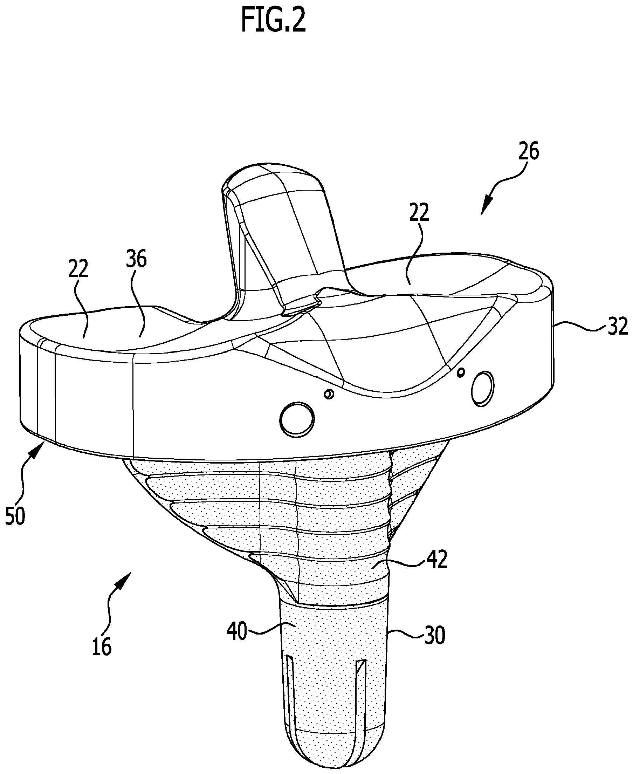

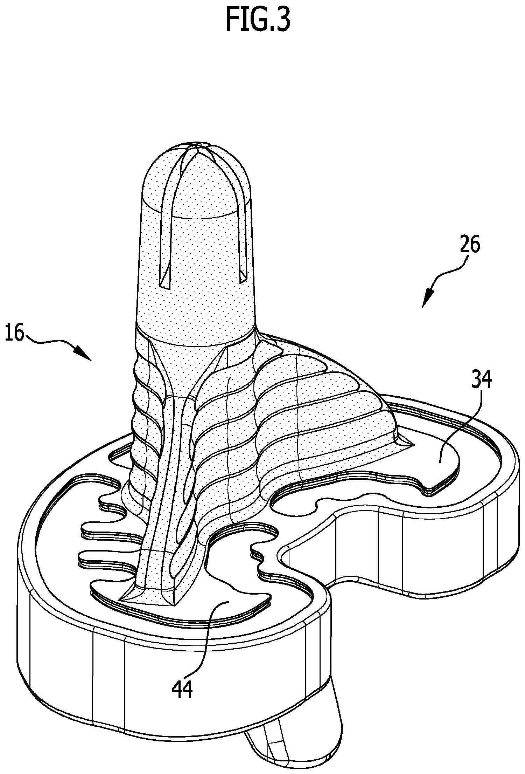

In Verbindung mit den Figuren 1 bis 3 wird der Aufbau des Implantats 26 in Form der Tibiakomponente 16 näher erläutert.

Das Implantat 26 umfasst einen in eine Knochenkavität 28 einführbaren Schaft 30, welcher aus einem Kunststoff ausgebildet ist. Ferner umfasst das Implantat 26 einen Grundkörper 32, von dessen Unterseite 34 sich der Schaft 30 weg erstreckt.

Die Gelenkflächen 22 sind auf einer Oberseite 36 des Grundkörpers 32 ausgebildet.

Eine äußere Oberfläche 38 des Schafts 30 definiert eine Knochenkontaktfläche 40. Diese ist mit einer ersten körperverträglichen Knochenkontaktschicht 42 versehen oder beschichtet oder trägt eine solche Knochenkontaktschicht 42.

Der Grundkörper 32 weist ferner eine Grundkörperknochenkontaktfläche 44 auf zum Anlegen an eine präparierte Knochenfläche 46. Beispielsweise kann es sich bei der Knochenfläche 46 wie schematisch in Figur 1 dargestellt um eine ebene, nach einer teilweisen Resektion einer Tibia 48 ausgebildete Oberfläche derselben handeln.

Die Grundkörperknochenkontaktfläche 44 bildet den verbleibenden Bereich der Unterseite 34, die den Schaft 30 umgibt.

Das Implantat 26 ist insgesamt einstückig ausgebildet, und zwar umfasst es einen aus einem Kunststoff monolithisch, beispielsweise durch Spritzgießen, gebildeten Implantatgrundkörper 50,.

Bei dem Kunststoff kann es sich insbesondere um einen körperunverträglichen Kunststoff handeln, der bei einem Knochenkontakt die eingangs beschriebenen unerwünschten Auswirkungen zur Folge hat. Bei dem nicht körperverträglichen Kunststoff kann es sich insbesondere um Polyethylen oder Polyethylen mit ultrahohem Molekulargewicht (UHMWPE) handeln.

Der monolithische Implantatgrundkörper 50 ist wie beschrieben nur im Bereich des Schafts 30 mit der körperverträglichen Knochenkontaktschicht 42 versehen. Diese weist eine Dicke in einem Bereich von etwa 1 µm bis etwa 1000 µm auf. Insbesondere kann sie eine Dicke in einem Bereich von etwa 5 µm bis etwa 700 µm aufweisen.

Die erste Knochenkontaktschicht 42 weist ferner eine Rauheit auf. Diese kann in einem Bereich von etwa 0,5 µm bis etwa 10 µm liegen.

Die Knochenkontaktfläche 40, also ein Teil der äußeren Oberfläche des Implantatgrundkörpers 50, kann ebenfalls rau sein und eine Rauheit in einem Bereich von etwa 0,5 µm bis etwa 10 µm aufweisen.

Die erste Knochenkontaktschicht 42 kann beispielsweise aus einem Metall, insbesondere aus Titan oder einer Titan enthaltenden Legierung oder aus einem körperverträglichen Kunststoff wie beispielsweise Polyetheretherketon ausgebildet sein. Alternativ kann die Knochenkontaktschicht 42 auch aus einer Keramik oder aus einer knochenmineralischen Substanz ausgebildet sein. Die knochenmineralische Substanz kann insbesondere Hydroxylapatit sein.

Die erste Knochenkontaktschicht 42 kann insbesondere durch Kaltgasspritzen und/oder durch physikalische und/oder durch chemische Gasphasenabscheidung ausgebildet sein.

Zur Vermeidung eines Kontakts zwischen dem Implantatgrundkörper 50 und dessen Knochenkontaktfläche 40 mit Knochengewebe ist die erste Knochenkontaktschicht 42 vollständig geschlossen ausgebildet.

Die Grundkörperknochenkontaktfläche 44 ist unbeschichtet. Zur Vermeidung eines Kontakts mit Knochengewebe kann hier zum Befestigen des Implantats 26 Knochenzement zum Einsatz kommen. Der Knochenzement bildet dann eine Trennschicht zwischen der Grundkörperknochenkontaktfläche 44 und dem Knochengewebe und dient zudem zur Fixierung des Implantats 26 am Knochen.

Die monolithische Ausbildung des Implantatgrundkörpers 50 hat den Vorteil, dass die Gelenkfläche 22 aus dem insbesondere körperunverträglichen Kunststoff ausgebildet werden kann, aus dem der Implantatgrundkörper 50 gebildet ist.

Das in den Figuren 1 bis 3 beispielhaft dargestellte Ausführungsbeispiel des Implantats 26 ist derart ausgebildet, dass der Schaft 30 bestimmungsgemäß ohne Knochenzement in der Knochenkavität 28 verankerbar ist. Dies wird ermöglicht durch die Knochengewebe nicht angreifende Knochenkontaktschicht 42.

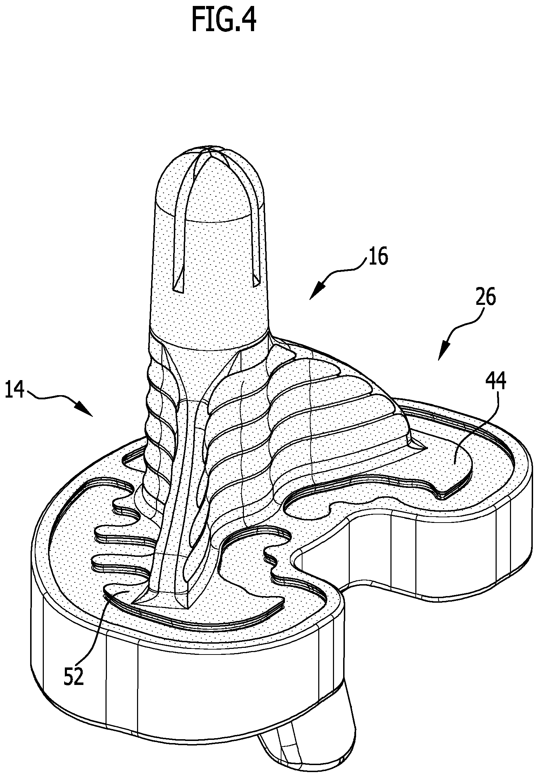

Ein weiteres Ausführungsbeispiel eines Implantats 26 in Form einer ersten Gelenkkomponente 14, die als Tibiakomponente 16 ausgebildet ist, ist schematisch in Figur 4 dargestellt. Dieses Ausführungsbeispiel unterscheidet sich vom in den Figuren 1 bis 3 dargestellten Ausführungsbeispiel des Implantats 26 lediglich dadurch, dass die Grundkörperknochenkontaktfläche 44 ebenfalls beschichtet ist, und zwar mit einer zweiten Knochenkontaktschicht 52.

Die zweite Knochenkontaktschicht 52 kann insbesondere identisch ausgebildet sein wie die erste Knochenkontaktschicht 42. Auf die oben angegebenen Eigenschaften der ersten Knochenkontaktschicht 42 wie Dicke, Rauheit sowie Art der Herstellung sei hier zur Vermeidung von Wiederholungen verwiesen.

Die zweite Knochenkontaktschicht 52 kann auch aus den oben beschriebenen Materialien ausgebildet sein, die im Zusammenhang mit der Beschreibung der Knochenkontaktschicht 42 angegeben sind.

Das Vorsehen der zweiten Knochenkontaktschicht 52 ermöglicht es insbesondere, den Grundkörper 32 bestimmungsgemäß ohne Knochenzement am Knochen, also insbesondere an der Knochenfläche 46, zu verankern.

Der Schaft 30 kann optional bei beiden beschriebenen Ausführungsbeispielen des Implantats 26 auch derart ausgebildet sein, dass er bei Bedarf alternativ oder zusätzlich mit Knochenzement im Knochen verankerbar ist.

Die Femurkomponente 20 kann in ähnlicher Weise wie die Tibiakomponente 16 einen Schaft aufweisen zum Verankern in einer Knochenkavität eines Femurs. Auch hier können der Schaft und gegebenenfalls ein Grundkörper der Femurkomponente 20 mit einer oder mehreren Knochenkontaktschichten versehen sein, um bei einer Implantation der Femurkomponente am Femur ohne Einsatz von Knochenzement einen direkten Kontakt zwischen dem Material, aus dem ein Implantatgrundkörper der Femurkomponente ausgebildet ist, umliegendem Knochengewebe zu vermeiden.

### Bezugszeichenliste

- 10: Gelenkimplantat
- 12: Kniegelenkendoprothese
- 14: erste Gelenkkomponente
- 16: Tibiakomponente
- 18: zweite Gelenkkomponente
- 20: Femurkomponente
- 22: Gelenkfläche
- 24: Gelenkfläche
- 26: Implantat
- 28: Knochenkavität
- 30: Schaft
- 32: Grundkörper
- 34: Unterseite
- 36: Oberseite
- 38: Oberfläche
- 40: Knochenkontaktfläche
- 42: erste Knochenkontaktschicht
- 44: Grundkörperknochenkontaktfläche
- 46: Knochenfläche
- 48: Tibia
- 50: Implantatgrundkörper
- 52: zweite Knochenkontaktschicht

## Patentansprüche

1. Implantat (26) mit einem in eine Knochenkavität (28) einführbaren Schaft (30), welcher Schaft aus einem Kunststoff, insbesondere aus einem körperunverträglichen Kunststoff, ausgebildet ist und mindestens eine Knochenkontaktfläche (40) definiert, wobei die Knochenkontaktfläche (40) mit einer ersten körperverträglichen Knochenkontaktschicht (42) versehen oder beschichtet ist oder eine körperverträgliche Knochenkontaktschicht (42) trägt, **dadurch gekennzeichnet, dass** der Schaft (30) des Implantats (26) bestimmungsgemäß ohne Knochenzement in der Knochenkavität (28) verankerbar ist und dass die erste Knochenkontaktschicht (42) vollständig geschlossen ausgebildet ist.

2. Implantat nach Anspruch 1, **dadurch gekennzeichnet, dass** das Implantat (26) einen Grundkörper (32) umfasst und dass sich der Schaft (30) von einer Unterseite (34) des Grundkörpers (32) weg erstreckt.

3. Implantat nach Anspruch 2, **dadurch gekennzeichnet, dass** der Grundkörper (32) eine Grundkörperknochenkontaktfläche (44) aufweist zum Anlegen an eine präparierte Knochenfläche (46).

4. Implantat nach Anspruch 3, **dadurch gekennzeichnet, dass** die Grundkörperknochenkontaktfläche (44) mit einer zweiten körperverträglichen Knochenkontaktschicht (52) versehen ist oder eine zweite körperverträgliche Knochenkontaktschicht (52) trägt,
wobei insbesondere die zweite Knochenkontaktschicht (52) vollständig geschlossen ausgebildet ist.

5. Implantat nach einem der Ansprüche 2 bis 4, **dadurch gekennzeichnet, dass** der Grundkörper (32) bestimmungsgemäß ohne Knochenzement am Knochen verankerbar ist.

6. Implantat einem der voranstehenden Ansprüche, **dadurch gekennzeichnet, dass**
a) das Implantat (26) bestimmungsgemäß ohne Knochenzement am Knochen verankerbar ist
und/oder
b) die erste Knochenkontaktschicht (42) und die zweite Knochenkontaktschicht (52) identisch ausgebildet sind.

7. Implantat nach einem der voranstehenden Ansprüche, **dadurch gekennzeichnet, dass**
a) die erste und/oder die zweite Knochenkontaktschicht (42, 52) eine Dicke in einem Bereich von etwa 1 µm bis etwa 1000 µm aufweisen, insbesondere in einem Bereich von etwa 5 µm bis etwa 700 µm,
und/oder
b) die erste und/oder die zweite Knochenkontaktschicht (42, 52) eine Rauheit in einem Bereich von etwa 0,5 µm bis etwa 10 µm aufweisen
und/oder
c) die Knochenkontaktfläche (40) und/oder die Grundkörperknochenkontaktfläche (44) eine Rauheit in einem Bereich von etwa 0,5 µm bis etwa 10 µm aufweisen.

8. Implantat nach einem der voranstehenden Ansprüche, **dadurch gekennzeichnet, dass** die erste und/oder die zweite Knochenkontaktschicht (42, 52) aus mindestens einem Metall und/oder aus einem körperverträglichen Kunststoff und/oder aus einer Keramik und/oder aus mindestens einer knochenmineralischen Substanz ausgebildet sind.

9. Implantat nach Anspruch 8, **dadurch gekennzeichnet, dass**
a) das mindestens eine Metall Titan ist oder enthält und/oder
b) der körperverträgliche Kunststoff Polyetheretherketon (PEEK) ist oder enthält
und/oder
c) die knochenmineralische Substanz Hydroxylapatit ist.

10. Implantat nach einem der voranstehenden Ansprüche, **dadurch gekennzeichnet, dass** die erste und/oder die zweite Knochenkontaktschicht (52) durch Kaltgasspritzen und/oder durch physikalische und/oder durch chemische Gasphasenabscheidung ausgebildet sind.

11. Implantat nach einem der voranstehenden Ansprüche, **dadurch gekennzeichnet, dass**
a) das Implantat (26) bis auf die erste und/oder die zweite Knochenkontaktschicht vollständig aus dem körperunverträglichen Kunststoff ausgebildet ist
und/oder
b) der nicht körperverträgliche Kunststoff Polyethylen oder ultrahochmolekulares Polyethylen ist
und/oder
c) das Implantat (26) einstückig, insbesondere monolithisch, ausgebildet ist.

12. Implantat nach einem der voranstehenden Ansprüche, **dadurch gekennzeichnet, dass** das Implantat (26) in Form einer ersten Gelenkkomponente (14; 18) eines künstlichen Gelenkimplantats (10) ausgebildet ist und mindestens eine Gelenkfläche (22; 24) aufweist.

13. Implantat nach Anspruch 12, **dadurch gekennzeichnet, dass**
a) die mindestens eine Gelenkfläche (22; 24) durch den körperunverträglichen Kunststoff gebildet ist
und/oder
b) die erste Gelenkkomponente (14) in Form einer Tibiakomponente (16) einer Kniegelenkendoprothese (12) ausgebildet ist.

14. Gelenkimplantat (10) mit einer ersten Gelenkkomponente (14) und mindestens einer zweiten, mit der ersten Gelenkkomponente (14) zusammenwirkenden Gelenkkomponente (18), **dadurch gekennzeichnet, dass** die erste und/oder die mindestens eine zweite Gelenkkomponente (14, 18) in Form eines Implantats (26) nach einem der voranstehenden Ansprüche ausgebildet sind.

15. Gelenkimplantat nach Anspruch 14, **dadurch gekennzeichnet, dass** das Gelenkimplantat(10) in Form einer Kniegelenkendoprothese (12) ausgebildet ist, dass die erste Gelenkkomponente (14) in Form einer Tibiakomponente (16) und dass die zweite Gelenkkomponente (18) in Form einer Femurkomponente (20) ausgebildet ist.

## Claims

1. Implant (26) with a shank (30) which is insertible into a bone cavity (28), which shank is made of a plastic, in particular of a bioincompatible plastic, and defines at least one bone contact face (40), wherein the bone contact face (40) is provided or coated with a first biocompatible bone contact layer (42) or bears a biocompatible bone contact layer (42), **characterized in that** the shank (30) of the implant (26) is intended to be anchored in the bone cavity (28) without bone cement and **in that** the first bone contact layer (42) is formed entirely closed.

2. Implant in accordance with Claim 1, **characterized in that** the implant (26) comprises a base body (32) and **in that** the shank (30) extends away from a lower side (34) of the base body (32).

3. Implant in accordance with Claim 2, **characterized in that** the base body (32) has a base body bone contact face (44) for abutting against a prepared bone face (46).

4. Implant in accordance with Claim 3, **characterized in that** the base body bone contact face (44) is provided with a second biocompatible bone contact layer (52) or bears a second biocompatible bone contact layer (52),
wherein, in particular the second bone contact layer (52) is formed entirely closed.

5. Implant in accordance with any one of Claims 2 to 4, **characterized in that** the base body (32) is intended to be anchored on the bone without bone cement.

6. Implant in accordance with any one of the preceding Claims, **characterized in that**
a) the implant (26) is intended to be anchored on the bone without bone cement
and/or
b) the first bone contact layer (42) and the second bone contact layer (52) are of identical configuration.

7. Implant in accordance with any one of the preceding Claims, **characterized in that**
a) the first and/or the second bone contact layer (42, 52) have a thickness in a range of about 1 µm to about 1000 µm, in particular in a range of about 5 µm to about 700 µm
and/or
b) the first and/or the second bone contact layer (42, 52) have a roughness in a range of about 0.5 µm to about 10 µm
and/or
c) the bone contact face (40) and/or the base body bone contact face (44) have a roughness in a range of about 0.5 µm to about 10 µm.

8. Implant in accordance with any one of the preceding Claims, **characterized in that** the first and/or the second bone contact layer (42, 52) are made of at least one metal and/or of a biocompatible plastic and/or of a ceramic and/or of at least one bone mineral substance.

9. Implant in accordance with Claim 8, **characterized in that**
a) the at least one metal is or contains titanium
and/or
b) the biocompatible plastic is or contains polyetheretherketone (PEEK)
and/or
c) the bone mineral substance is hydroxyapatite.

10. Implant in accordance with any one of the preceding Claims, **characterized in that** the first and/or the second bone contact layer (52) are formed by cold gas spraying and/or by physical and/or by chemical vapor deposition.

11. Implant in accordance with any one of the preceding Claims, **characterized in that**
a) the implant (26), except for the first and/or the second bone contact layer, is made entirely of the bioincompatible plastic
and/or
b) the bioincompatible plastic is polyethylene or ultra high molecular weight polyethylene
and/or
c) the implant (26) is formed as one piece, in particular monolithically.

12. Implant in accordance with any one of the preceding Claims, **characterized in that** the implant (26) is configured in the form of a first joint component (14; 18) of an artificial joint implant (10) and has at least one joint face (22; 24).

13. Implant in accordance with Claim 12, **characterized in that**
a) the at least one joint face (22; 24) is formed by the bioincompatible plastic
and/or
b) the first joint component (14) is configured in the form of a tibial component (16) of a knee joint endoprosthesis (12).

14. Joint implant (10) with a first joint component (14) and at least one second joint component (18) cooperating with the first joint component (14), **characterized in that** the first and/or the at least one second joint component (14, 18) are configured in the form of an implant (26) in accordance with any one of the preceding Claims.

15. Joint implant in accordance with Claim 14, **characterized in that** the joint implant (10) is configured in the form of a knee joint endoprosthesis (12), **in that** the first joint component (14) is configured in the form of a tibial component (16), and **in that** the second joint component (18) is configured in the form of a femoral component (20).

## Revendications

1. Implant (26) avec une tige (30) qui peut être insérée dans une cavité d'os (28), laquelle tige est réalisée en plastique, en particulier en plastique incompatible avec le corps, et définit au moins une surface de contact avec un os (40), où la surface de contact avec un os (40) est munie ou revêtue d'une première couche de contact avec un os compatible avec le corps (42) ou porte une couche de contact avec un os compatible avec le corps (42), **caractérisé en ce que** la tige (30) de l'implant (26) peut être ancrée dans la cavité d'os (28) conformément à la destination sans ciment osseux et **en ce que** la première couche de contact avec un os (42) est réalisée de manière totalement fermée.

2. Implant selon la revendication 1, **caractérisé en ce que** l'implant (26) comprend un corps de base (32) et **en ce que** la tige (30) s'étend à distance depuis un côté inférieur (34) du corps de base (32).

3. Implant selon la revendication 2, **caractérisé en ce que** le corps de base (32) présente une surface de contact avec un os de corps de base (44) pour l'appui contre une surface d'os préparée (46).

4. Implant selon la revendication 3, **caractérisé en ce que** la surface de contact avec un os de corps de base (44) est munie d'une seconde couche de contact avec un os compatible avec le corps (52) ou porte une seconde couche de contact avec un os compatible avec le corps (52), où en particulier la seconde couche de contact avec un os (52) est réalisée de manière totalement fermée.

5. Implant selon l'une des revendications 2 à 4, **caractérisé en ce que** le corps de base (32) peut être ancré à l'os sans ciment osseux conformément à la destination.

6. Implant selon l'une des revendications précédentes, **caractérisé en ce que**
a) l'implant (26) peut être ancré à l'os sans ciment osseux conformément à la destination
et/ou
b) la première couche de contact avec un os (42) et la seconde couche de contact avec un os (52) sont réalisées de manière identique.

7. Implant selon l'une des revendications précédentes, **caractérisé en ce que**
a) la première et/ou la seconde couche de contact avec un os (42, 52) présentent une épaisseur dans une plage d'environ 1 µm à environ 1000 µm, en particulier dans une plage d'environ 5 µm à environ 700 µm,
et/ou
b) la première et/ou la seconde couche de contact avec un os (42, 52) présentent une rugosité dans une plage d'environ 0,5 µm à environ 10 µm
et/ou
c) la surface de contact avec un os (40) et/ou la surface de contact avec un os de corps de base (44) présentent une rugosité dans une plage d'environ 0,5 µm à environ 10 µm.

8. Implant selon l'une des revendications précédentes, **caractérisé en ce que** la première et/ou la seconde couche de contact avec un os (42, 52) sont réalisées en au moins un métal et/ou un plastique compatible avec le corps et/ou une céramique et/ou au moins une substance minérale osseuse.

9. Implant selon la revendication 8, **caractérisé en ce que**
a) le au moins un métal est ou contient du titane et/ou
b) le plastique compatible avec le corps est ou contient de la polyétheréthercétone (PEEK)
et/ou
c) la substance minérale osseuse est l'hydroxylapatite.

10. Implant selon l'une des revendications précédentes, **caractérisé en ce que** la première et/ou la seconde couche de contact avec un os (52) est réalisée par projection de gaz froid et/ou par dépôt physique et/ou dépôt chimique en phase vapeur.

11. Implant selon l'une des revendications précédentes, **caractérisé en ce que**
a) l'implant (26) est entièrement réalisé en le plastique non compatible avec le corps y compris la première et/ou la seconde couche de contact avec un os
et/ou
b) le plastique non compatible avec le corps est le polyéthylène ou le polyéthylène à ultra haut poids moléculaire
et/ou
c) l'implant (26) est réalisé en une seule pièce, en particulier monolithique.

12. Implant selon l'une des revendications précédentes, **caractérisé en ce que** l'implant (26) est réalisé sous forme d'un premier composant d'articulation (14 ; 18) d'un implant d'articulation artificiel (10) et présente au moins une surface d'articulation (22 ; 24).

13. Implant selon la revendication 12, **caractérisé en ce que**
a) la au moins une surface d'articulation (22 ; 24) est formée par le plastique incompatible avec le corps
et/ou
b) le premier composant d'articulation (14) est réalisé sous forme d'un composant tibia (16) d'une endoprothèse d'articulation du genou (12).

14. Implant d'articulation (10) avec un premier composant d'articulation (14) et au moins un second composant d'articulation (18) coopérant avec le premier composant d'articulation (14), **caractérisé en ce que** le premier et/ou le au moins un second composant d'articulation (14, 18) sont réalisés sous forme d'un implant (26) selon l'une des revendications précédentes.

15. Implant d'articulation selon la revendication 14, **caractérisé en ce que** l'implant d'articulation (10) est réalisé sous forme d'une endoprothèse d'articulation du genou (12), **en ce que** le premier composant d'articulation (14) est réalisé sous forme d'un composant tibia (16) et **en ce que** le second composant d'articulation (18) est réalisé sous forme d'un composant fémur (20).
